# EUROPEAN PATENT SPECIFICATION

(11) **EP 4 475 814 B1**
(45) Date of publication and mention of the grant of the patent: **06.08.2025**
(21) Application number: 23702152.2
(22) Date of filing: 31.01.2023
(51) Int. Cl.: A61K 8/365, A61K 8/41, A61Q 19/02, A61Q 19/10

(54) **A SKIN BRIGHTENING COMPOSITION**
HAUTAUFHELLUNGSZUSAMMENSETZUNG
COMPOSITION POUR ÉCLAIRCIR LA PEAU

(30) Priority: 09.02.2022 EP 22155751
(43) Date of publication of application: 18.12.2024
(73) Proprietor: Unilever IP Holdings B.V., 3013 AL Rotterdam (NL); Unilever Global IP Limited, Wirral, Merseyside CH62 4ZD (GB)
(72) Inventor: DAMODARAN, Anita, 6708 WH Wageningen (NL); MATHAPATHI, Mruthyunjaya Swamy, 6708 WH Wageningen (NL); VENKATESH, Satish Kumar, 6708 WH Wageningen (NL)
(74) Representative: James, Helen Sarah
(86) International application number: PCT/EP2023/052309
(87) International publication number: WO 2023/151986

(56) References cited:
- CN-A- 108 175 712
- CN-A- 108 371 647
- US-A1- 2018 369 092
- DATABASE GNPD [online] MINTEL; 16 July 2013 (2013-07-16), ANONYMOUS: "Lip Balm", XP055944941, retrieved from https://www.gnpd.com/sinatra/recordpage/2119069/ Database accession no. 2119069

## Description

### Field of the Invention

The present invention relates to a personal care composition, more particularly a topical composition which is effective in reducing melanin synthesis which leads to brightening skin.

### Background of the Invention

Most people consider skin appearance especially on the face as one of the key indicators of their own beauty and health. Having an even skin tone which is bright and free of blemishes is thus desired by many. The degree and evenness of pigmentation of the skin is affected by factors like age, hormonal changes, occurrence of acne and exposure to sunlight and pollution. These can cause spots or freckles, or hyper-pigmentation on certain localised areas of skin like under-eye dark circles. Many people believe that certain life-style factors like hydration (quantity of water consumed), the type of food and quantity and quality of sleep also has an effect on skin appearance. Since people are aware that changes like leading a healthier lifestyle may take a long time for evening out skin appearance, they rely on cosmetic solution to give them temporary solution to blemishes on their skin. They also seek such products to reduce the skin darkening caused by exposure to sunlight. To meet this need, many attempts have been made to develop products that reduce pigment production in the melanocytes.

The present inventors have been working in this area for a long time and have also filed several patent applications and produced various cosmetic products which are in the market. In the present invention, they were specifically looking for cost effective solution to this problem which could also be attained using commonly used cosmetic ingredients for delivering even and bright skin tone.

They are aware that certain hydroxystearic acid (HSA) e.g. 12-hydroxystearic acid provide skin lightening benefit e.g. WO09153169 which discloses skin lightening additives and skin lightening compositions having at least one of a hetero substituted saturated or unsaturated aliphatic acid are described. The compositions disclosed therein were found to be suitable for topical application and may comprise 12-hydroxystearic acid, ricinoleic acid or both. US2018/369092 is directed to a cosmetic composition comprising a hydroxy functionalized solvent and 12-hydroxystearic acid.

The present inventors during the course of their extensive experimentation in this area were looking for certain commonly available and inexpensive actives which would interact with HSA to deliver enhanced and even synergistic improvement in reducing melanin on skin. They hit upon tetrahydoxypropyl ethylenediamine (THPE) which is generally used as a chelating agent which they found to deliver enhanced efficacy in combination with HSA. THPE has been reported to be a skin darkening agent e.g in US2011/0081305 which discloses a composition comprising a skin-lightening resorcinol and a skin darkening agent. The composition disclosed therein may be topically applied to skin to treat signs of aging.

It is thus an object of the present invention to provide for a solution to hyperpigmentation, uneven skin appearance, sun-induced dark spots thereby delivering a bright and even skin appearance.

It is yet another object of the present invention to provide for such a solution which is cost effective.

### Summary of the Invention

The first aspect of the present invention relates to a skin brightening composition comprising
(i) 0.1 to 4.0% tetrahydroxypropyl ethylenediamine;
(ii) hydroxystearic acid preferably 12-hydroxystearic acid; and
(iii) a cosmetically acceptable carrier.

Another aspect of the present invention relates to a method of providing brightness to skin comprising the step of applying a composition as claimed in any one of the preceding claims on to skin.

### Detailed Description of the Invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The topical composition of the invention is meant to be used for personal care or for cosmetic use and could also be referred to as a personal care composition or a cosmetic composition. By a "personal care composition" as used herein, is meant to include a composition for topical application i.e external surfaces of the skin and/or hair of humans. Such a composition may be classified to be of the leave-on type or of the wash off type, and includes any product applied to a human body for improving appearance, cleansing, odour control or general aesthetics. The composition is preferably of the leave-on type. The composition of the present invention in a leave-on type can be in the form of a gel, emulsion, lotion or cream. Preferred composition is a cream. The composition could also be in a wash-off type i.e those compositions which are used to cleanse the skin. Such compositions are generally high in surfactants. They are usually applied on the skin or hair generally diluted with large amount of water and are accompanied with high amount of foam. The composition is then rinsed off with copious amounts of water during which action the oils and dirt removed from the clean micellize in the rinse water and leave a cleaner skin surface than before the cleaning action.

"Skin" as used herein is meant to include skin on the face and body (e.g., neck, chest, back, arms, underarms, hands, legs and scalp) and especially to the exposed parts thereof.

The composition of the invention comprises tetrahydroxypropyl ethylenediamine (THPE) which has the formula

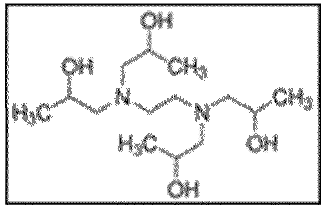

THPE is included in 0.1 to 4.0%, preferably 0.5 to 2%, by weight of the composition.

The composition of the invention comprises hydroxystearic acid. It is preferred that the hydroxystearic acid is 10-hydroxystearic acid, 12-hydroxystearic acid or trihydoxystearic acid (e.g. 9,10,13-trihydroxystearic acid) or trihydroxy stearin or compounds that yield one or more molecules of hydroxystearic acid or hydroxystearate on their breakdown like mono, di or tri ester of glycerol with hydroxystearic acid. Of these, 10-hydroxystearic acid, 12-hydroxystearic acid and 9,10,13-trihydroxystearic acid are more preferred, 12-hydroxystearic acid (12-HSA) being most preferred. 12-HSA has the structure as given below:

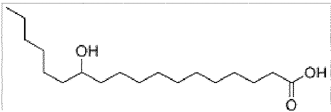

Hydroxystearic acid is preferably included in 0.1 to 5%, more preferably 0.2 to 4%, further more preferably 0.5 to 2% by weight of the composition.

The composition preferably includes niacinamide. Niacinamide also known as nicotinamide and as pyridine-3-carboxamide is the active, water soluble form of vitamin B3. When included the composition of the present invention comprises an effective amount of niacinamide, typically in a concentration of 0.1 to 3%, more preferably from 0.5 to 2% by weight of the composition.

The composition may comprise an organic sunscreen selected from one or both of a UVA sunscreen and a UVB sunscreen. Sunscreens include those materials commonly employed to block ultraviolet light. Illustrative compounds are the derivatives of PABA, cinnamate and salicylate. For example, avobenzophenone (Parsol 1789^{®}) octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks, Parsol MCX and Benzophenone-3, respectively. The exact amount of sunscreen employed in the compositions can vary depending upon the degree of protection desired from the sun's UV radiation. Additives that reflect or scatter the sun rays may also be employed. These additives include oxides like zinc oxide and titanium dioxide.

The composition of the present invention may further comprise a cosmetically acceptable carrier, which may act as diluents, dispersants. and/or carriers for the actives used in the composition, so as to facilitate their distribution when the composition is applied to the skin. The cosmetically acceptable vehicle suitable for use in the present invention may be aqueous, anhydrous or an emulsion; aqueous or an emulsion, especially water-in-oil or oil-in-water emulsion being most preferred. Water when present typically makes up the balance of the composition. Preferably water is present in a concentration of 5 to 99%, more preferably from 20 to 80%, still more preferably from 40 and 80% by weight of the composition.

### Leave on compositions

The composition of the present invention in a leave-on type may be delivered in a cream, lotion or gel form preferably in cream form. A preferred format for the solid form of the composition is a cream, further more preferably one which has a vanishing cream base. Vanishing cream base is one which comprises 3 to 25 wt% fatty acid. Optionally, the composition may comprise 0.1 to 10 wt% soap. When included, the fatty acid is preferably a C10 to C22 fatty acid, more preferably a C16 to C18 fatty acid. Most preferably the fatty acids are stearic acid or palmitic acid or a mixture thereof and the soap is preferably the potassium salt of the fatty acid mixture. The fatty acid is often hystric acid which is substantially (generally about 90 to 95 %) a mixture of 45 % stearic acid and 55 % palmitic acid. The most preferred cream is one having 3 to 25 wt% fatty acid and 0.1 to 10 wt% soap.

Preferably, the composition comprises emollients. Examples of emollients that may be used in the leave-on composition include stearyl alcohol, glyceryl monoricinoleate, mink oil, isopropyl isostearate, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, din-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isopropyl linoleate, lauryl lactate, myristyl lactate, decyl oleate, myristyl myristate and mixtures thereof.

Preferably, the composition comprises solvents. Examples of solvents that may be used in the composition include ethyl alcohol, isopropanol, acetone, ethylene glycol mono ethyl ether, diethylene glycol mono butyl ether, diethylene glycol mono ethyl ether and mixtures thereof. The composition may comprise polyhydric alcohols which may be selected from one or more of glycerine, 1,3-butylene glycol, propylene glycol, 1,3-propanediol, pentylene glycol, hexylene glycol, and sorbitol.

Preferably, the composition comprises powders. Examples of powders that may be used in the composition include chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate and mixtures thereof.

Preferably, the composition comprises preservatives to protect against the growth of potentially harmful microorganisms. Examples of ingredients that may be used as preservatives in the composition include alkyl esters of para-hydroxybenzoic acid, hydantoin derivatives, propionate salts, and a variety of quaternary ammonium compounds. More preferably, ingredients that may be used as preservative in the composition are sodium benzoate, iodopropynyl butyl carbamate, methylisothiazolinone, iodopropynylbutylcarbamate, phenoxyethanol, methyl paraben, propyl paraben, imidazolidinyl urea, sodium dehydroacetate, ethylhexylglycerin, benzyl alcohol, alkane diols and mixtures thereof. When present in the composition, preservatives are added preferably in an amount 0.001 to 5 wt%, more preferably 0.01 to 3 wt% and most preferably 0.02 to 2 wt%, even most preferably 0.25 to 1.5%.

Preferably, the composition comprises a range of other optional ingredients that include antioxidants, binders, buffering agents, colorants, astringents, fragrance, opacifying agents, conditioners, exfoliating agents, pH adjusters, skin sensates, skin soothing agents, and skin healing agents.

The packaging for the composition of this invention can be a patch, bottle, tube, roll-ball applicator, propellant driven aerosol device, squeeze container or lidded jar.

### Wash-off compositions

The composition of the present invention when delivered for wash-off purposes can be in the form of a liquid, lotion, cream, foam, scrub, gel, shampoo, conditioner, handwash, facewash or bodywash product.

The cosmetically acceptable carrier in this case generally comprises water in addition to surfactant. A particularly preferred surfactant is an anionic surfactant like soap. The soap for preparing the cleansing composition of the invention is preferably a C₈-C₂₄ soap, more preferably C₁₀-C₂₀ soap and most preferably C₁₂-C₁₈ soap. The cation of the soap can be alkali metal, alkaline earth metal or ammonium. Preferably, the cation of the soap is selected from sodium, potassium or ammonium. More preferably the cation of the soap is sodium or potassium. Fatty acids derived from other suitable oils/fats such as groundnut, soybean, tallow, palm, palm kernel, etc. may also be used in other desired proportions. A cosmetically acceptable carrier in this format generally forms 80 to 99% by weight of the cleansing composition.

When present, the anionic surfactant e.g. soap, is preferably present in an amount of 1 to 90%, preferably from 10 to 85%, more preferably 25 to 75% by weight of the cleansing composition. The cleansing composition is preferably in the form of a solid or semi solid form, most preferably in a solid form. Preferred solid compositions are in the shape of a soap bar.

Other anionic surfactants are preferably selected from alkyl ether sulphate, primary alkyl sulphate, secondary alkyl sulphonates, alkyl benzene sulphonates, or ethoxylated alkyl sulphates. The anionic surfactant other than soap which is preferred in the cleansing composition is an alkyl ether sulphate preferably those having between 1 and 3 ethylene oxide groups, either from natural or synthetic source and/or sulphonic acid. Especially preferred are sodium lauryl ether sulphates. Alkyl polyglucoside may also be present in the composition, preferably those having a carbon chain length between C₆ and C₁₆.

Preferred cleansing compositions may include other known ingredients such as perfumes, pigments, preservatives, emollients, sunscreens, gelling agents and thickening agents. Choice of these ingredients will largely depend on the format of the composition. Water is a preferred carrier. When water is present, it is preferably present in at least 1%, more preferably at least 2%, furthermore preferably at least 5% by weight of the composition. When water is the carrier, a preferred cleansing composition comprises 10 to 50%, more preferably 12 to 40%, further more preferably from 12 to 25% by weight water.

The cleansing composition of the invention may also be delivered through a moisturizing bar or a moisturizing liquid composition. Moisturizing bar compositions comprising fatty acyl isethionates (e.g. cocyl isethionate) are especially preferred.

Fatty acyl isethionates (e.g., cocoyl isethionates) surfactant "products" are defined as mixtures of anionic acyl isethionate surfactants and fatty acids/fatty acid soaps. They are highly desirable in personal care skin or hair cleansing products, particularly in personal care products, because they lather well, are mild to the skin and have good emollient properties. Typically, fatty acid isethionate surfactant products are produced by esterification of fatty acids or by reaction of fatty acid chloride having carbon chain length of C8 to C20 with isethionate. A typical surfactant product containing fatty acyl isethionate contains about 40 to 95 wt.% acid isethionate, and 5 to 50 wt.%, typically 10 to 40 wt.% free fatty acid, in addition to isethionate salts, typically at less than 5%, and trace (less than 2 wt.%) of other additives. Such compositions may also include an alkyl taurates, e.g., alkyl taurate amides. Such alkyl taurate amides may be made by reaction of a taurine; methyl taurine; or a corresponding taurate salt (e.g., NH2CH2CH2SO3 M+, where M+ may be sodium or potassium counterion) with the appropriate fatty acid. Preferably alkyl taurate amides include sodium methyl cocoyl taurate and sodium methyl lauroyl taurate. Fatty acid soap may be included in the range of 5 to 15 wt%. Other surfactants like betaines may be included in 1 to 5 wt%. Water is generally included in 2 to 8 wt% of the composition.

Additionally, the wash-off composition of the invention may comprise polyhydric alcohols. Polyhydric alcohols described as being useful in leave-on types hereinabove may also be used in wash-off formats.

In a second aspect, the invention relates to use of the composition according to the invention for skin brightening.

In a third aspect, the invention relates to a method of brightening the skin of a human, the method comprising the step of applying the composition according to the invention onto the skin.

The invention will now be illustrated by means of the following non-limiting examples.

### Examples

### Examples A-B, 1: Melanin inhibition from skin cleansing compositions.

Effect of inclusion of actives like 12HSA at 1.58% (Examples B) and inclusion of 12HSA at 1.58% + THPE at 2% (Example - 1) was studied over control formulation without either of the above two actives (Example- A). The base formulation (Example - A) is given in the table - 1 below:

**Table -1**

| Ingredient | Wt% |
|---|---|
| Myristic acid | 25.0 |
| Stearic acid | 3.5 |
| Palmitic acid | 1.0 |
| Lauric acid | 3.0 |
| Potassium hydroxide (90%) | 7.7 |
| Propylene glycol | 7.5 |
| Glycerin | 22.5 |
| Glycol distearate | 3.0 |
| Glyceryl monostearate | 1.0 |
| Decyl glucoside | 5.0 |
| Water and minors | To 100 |

The formulations A , B and 1 were tested for % inhibition of melanin using the protocol given below.

### 3D model cleansing Protocol:

Day-0: Episkin RHPE (skin type: VI) tissues were removed from the transport plate and placed into a new 24 well plate containing 450 µl of maintenance media (provided by Episkin).

Day-1: After 18 hours +/- of 2 hrs of incubation in 37°C 5% CO₂ incubator, tissues were removed from the plate and spent media was stored in -80°C. Tissues were transferred to a fresh 24 well plate containing 450 µl of growth media (provided by Episkin). Nylon mesh (provided by Episkin) was prepared for active application, formulations were diluted 1:1 in distilled water and approximately 2mg of the formulation was added using 1ml syringe over the mesh and applied evenly to cover the mesh uniformly. Mesh with formulation was placed on top of the tissue for 20 seconds. After 20 seconds, mesh was removed and tissues were washed with distilled water twice for 20 seconds and incubation was continued at 37°C, 5% CO₂ incubator for 48 h.

Day-3: Tissues were transferred to fresh 24 well plate containing 450 µl of fresh growth media. Then the application of formulations as outlined in day 1 was repeated.

Day-5: The steps as in Day 3 was repeated.

Day-7: Tissues were washed with distilled water and then progressed for melanin content estimation and MTT assay.

Melanin Content Estimation: Tissues were transferred to 1.5 ml Eppendorf tubes containing 150 µl of Solvable (Tissue and Gel Solubilizer 0.5 M-Packard Bioscience Co. Catalogue No. 6NE9100). Then tubes were Incubated at 60°C for 12-16 h with gentle shaking. After complete dissolution, tubes were kept in room temperature for 30 minutes. Then, the tubes were centrifuged at 1000 rpm for 10 minutes. Then 100 µl of the supernatant was transferred into a 384 well plate and spectrophotometric OD readings at 490nm was taken for measuring the melanin content in the samples.

The results in terms of % melanin inhibition is summarized in table - 2 below.

**Table - 2: Melanin inhibition rate**

| Example | Active (concentration, wt%) | % Melanin inhibition |
|---|---|---|
| A | Control formulation as per Table- 1 | 5 |
| B | Control formulation + 1.58% 12HSA | 10 |
| 1 | Control formulation + 1.58% 12HSA + 2% | 16 |

The data in the above table indicates that inclusion of 1.58% 12HSA and 2% THPE provides for vastly improved melanin inhibition over control sample.

### Example - C-F,2: Effect of inclusion of the 12HSA, THPE and niacinamide on melanin synthesis on melanocyte cells in vitro

Experiments were carried out on melanocyte cells using the actives as shown in Table -3 using the procedure given below;
1. Human primary melanocytes were seeded at a density of 5x10⁴ per well into a 24 well plate with fresh melanocyte growth media (MGM).
2. Post 24 hrs seeding, cells were treated with actives in fresh MGM media and incubated for 72 hrs at 5% CO₂/37°C incubator.
3. After 72 h of incubation, melanin estimation was done.

Melanin content estimation: Cell culture spent media was removed and cells were washed once with 200 µl of 1 x PBS. After wash, 120 µl/well of MCA (melanin content assay) reagent (10% DMSO in 1N NaOH) was added and incubated for 1 hr at 60° C in a shaker incubator. Then 100 µl of this lysate was transferred to a fresh 384 well plate and absorbance was measured spectrophotometric at 405 nm with a micro plate reader.

**Table - 3**

| Example | Ingredients | Melanin synthesis (% of control) |
|---|---|---|
| C | Control (untreated) | 100 |
| D | 0.001% THPE | 91 |
| E | 0.12% niacinamide | 97 |
| F | 0.00024% 12HSA | 98 |
| 2 | 0.001% THPE + 0.12% B3 + 0.00024% 12HSA | 68 |

The data in the table- 3 above indicates that the inclusion of niacinamide in addition to THPE and hydroxystearic acid provides for further enhanced melanin inhibition thereby indicative of delivering even pigmentation as well as overall brighter skin tone.

## Claims

1. A skin brightening composition comprising
(i) 0.1 to 4.0% tetrahydroxypropyl ethylenediamine;
(ii) hydroxystearic acid preferably 12-hydroxystearic acid; and
(iii) a cosmetically acceptable carrier.

2. A composition as claimed in claim 1 comprising 0.1 to 5% hydroxystearic acid.

3. A composition as claimed in claim 1 or 2 additionally comprising niacinamide.

4. A composition as claimed in claim 3 comprising 0.1 to 3% niacinamide.

5. A composition of the leave-on type as claimed in any one of the preceding claims wherein the cosmetically acceptable carrier is in gel, emulsion, lotion or cream form.

6. A composition as claimed in claim 5 in cream form.

7. A composition as claimed in claim 6 wherein the cream comprises 3 to 25 wt% fatty acid and 0.1 to 10 wt% soap.

8. A composition of the wash-off type as claimed in any one of preceding claims 1 to 4 wherein the cosmetically acceptable carrier comprises water and a surfactant.

9. A composition as claimed in any one of the preceding claims wherein the cosmetically acceptable carrier comprises a polyhydric alcohol.

10. A composition as claimed in claim 9 wherein the polyhydric alcohol is selected from one or more of glycerine, 1,3-butylene glycol, propylene glycol, 1,3-propanediol, pentylene glycol, hexylene glycol, and sorbitol.

11. A composition as claimed in any one of the preceding claims additionally comprising an organic sunscreen selected from one or both of a UVA sunscreen and a UVB sunscreen.

12. A method of providing brightness to skin comprising the step of applying a composition as claimed in any one of the preceding claims on to skin

## Patentansprüche

1. Hautaufhellungszusammensetzung, umfassend
(i) 0,1 bis 4,0% Tetrahydroxypropylethylendiamin;
(ii) Hydroxystearinsäure, vorzugsweise 12-Hydroxystearinsäure; und
(iii) einen kosmetisch akzeptablen Träger.

2. Zusammensetzung, wie im Anspruch 1 beansprucht, umfassend 0,1 bis 5% Hydroxystearinsäure.

3. Zusammensetzung, wie im Anspruch 1 oder 2 beansprucht, zusätzlich umfassend Niacinamid.

4. Zusammensetzung, wie im Anspruch 3 beansprucht, umfassend 0,1 bis 3% Niacinamid.

5. Zusammensetzung vom Leave-on-Typ, wie in einem der vorhergehenden Ansprüche beansprucht, wobei der kosmetisch akzeptable Träger in Gel-, Emulsions-, Lotions- oder Cremeform vorliegt.

6. Zusammensetzung, wie im Anspruch 5 beansprucht, in Creme-Form.

7. Zusammensetzung, wie im Anspruch 6 beansprucht, wobei die Creme 3 bis 25 Gew.-% Fettsäure und 0,1 bis 10 Gew.-% Seife umfasst.

8. Abwaschbare Zusammensetzung, wie in irgendeinem der vorhergehenden Ansprüche 1 bis 4 beansprucht, wobei der kosmetisch akzeptable Träger Wasser und ein Tensid umfasst.

9. Zusammensetzung, wie in irgendeinem der vorhergehenden Ansprüche beansprucht, wobei der kosmetische akzeptable Träger einen mehrwertigen Alkohol umfasst.

10. Zusammensetzung, wie im Anspruch 9 beansprucht, wobei der mehrwertige Alkohol aus einer oder mehreren der folgenden Verbindungen ausgewählt ist: Glycerin, 1,3-Butylenglykol, Propylenglykol, 1,3-Propandiol, Pentylenglykol, Hexylenglykol und Sorbit.

11. Zusammensetzung, wie in irgendeinem der vorhergehenden Ansprüche beansprucht, die außerdem ein organisches Sonnenschutzmittel umfasst, ausgewählt aus einem UVA-Sonnenschutzmittel und/oder einem UVB-Sonnenschutzmittel.

12. Verfahren zum Aufhellen der Haut, umfassend den Schritt des Auftragens einer Zusammensetzung, wie in irgendeinem der vorhergehenden Ansprüche beansprucht, auf die Haut.

## Revendications

1. Composition éclaircissante pour la peau comprenant
(i) 0,1 à 4,0 % de tétrahydroxypropyléthylènediamine;
(ii) de l'acide hydroxystéarique, de préférence de l'acide 12-hydroxystéarique; et
(iii) un véhicule acceptable du point de vue cosmétique.

2. Composition selon la revendication 1, comprenant 0,1 à 5 % d'acide hydroxystéarique.

3. Composition selon la revendication 1 ou 2, comprenant en outre du niacinamide.

4. Composition selon la revendication 3, comprenant 0,1 à 3 % de niacinamide.

5. Composition du type sans rinçage selon l'une quelconque des revendications précédentes, dans laquelle le véhicule acceptable du point de vue cosmétique est sous forme de gel, d'émulsion, de lotion ou de crème.

6. Composition selon la revendication 5, sous forme de crème.

7. Composition selon la revendication 6, dans laquelle la crème comprend 3 à 25 % en poids d'acide gras et 0,1 à 10 % en poids de savon.

8. Composition du type lavable selon l'une quelconque des revendications 1 à 4, dans laquelle le véhicule acceptable du point de vue cosmétique comprend de l'eau et un tensioactif.

9. Composition selon l'une quelconque des revendications précédentes, dans laquelle le véhicule acceptable du point de vue cosmétique comprend un alcool polyhydroxylé.

10. Composition selon la revendication 9, dans laquelle l'alcool polyhydroxylé est choisi parmi un ou plusieurs parmi la glycérine, le 1,3-butylèneglycol, le propylèneglycol, le 1,3-propanediol, le pentylèneglycol, l'hexylèneglycol et le sorbitol.

11. Composition selon l'une quelconque des revendications précédentes, comprenant en outre un écran solaire organique choisi parmi l'un ou les deux d'un écran solaire UVA et d'un écran solaire UVB.

12. Procédé pour donner de l'éclat à la peau, comprenant l'étape d'application d'une composition selon l'une quelconque des revendications précédentes sur la peau.
